Europäisches Patentamt

European Patent Office

Office européen des brevets

(19)

(11) Veröffentlichungsnummer: **0 004 857**

**A2**

(12) **EUROPÄISCHE PATENTANMELDUNG**

(21) Anmeldenummer: **79100500.2**

(22) Anmeldetag: **20.02.79**

(51) Int. Cl.²: **G 01 N 33/16**
**G 01 N 1/28, B 01 D 43/00**
**G 01 N 21/00**

(30) Priorität: **14.04.78 DE 2816229**

(43) Veröffentlichungstag der Anmeldung:
**31.10.79 Patentblatt 79/22**

(84) Benannte Vertragsstaaten:
**BE CH DE FR GB IT LU NL SE**

(71) Anmelder: **BOEHRINGER MANNHEIM GMBH**
**Sandhofer Strasse 112-132 Postfach 31 01 20**
**D-6800 Mannheim 31- Waldhof(DE)**

(72) Erfinder: **Klose, Sigmar, Dr.**
**Maxhöhe 17**
**D-8131 Berg(DE)**

(72) Erfinder: **Buschek, Herbert**
**Possenhofener Strasse 12**
**D-8130 Starnberg(DE)**

(72) Erfinder: **Schlumberger, Helmut**
**Kaiser-Heinrich-Strasse 23**
**D-8121 Polling(DE)**

(74) Vertreter: **Weickmann, Heinrich, Dipl.-Ing. et al,**
**Postfach 860 820 Möhlstrasse 22**
**D-8000 München 86(DE)**

(54) Verfahren und Mittel zur Beseitigung von Trübungen.

(57) Zur Beseitigung von Trübungen in durch Probenmaterial getrübten Reaktionsgemischen, insbesondere für photometrische Messung werden dem Reaktionsgemisch

a) eine oder mehrere niedermolekulare organische Verbindungen wie aromatische Alkohole oder Amine, die elektro-negative Substituenten tragen, z.B. Phenole oder Naphthole, welche mit ein oder mehreren Halogenatomen oder/und Hydroxylgruppen substituiert sind, und

b) ein oder mehrere ebenfalls für sich klar lösliche Detergentien, welche mit dem Zusatz a) einen weniger löslichen Komplex bilden, wie nichtionogene Polyäthylenoxidaddukte oder kationische Detergentien, insbesondere Alkyl-, Aralkyl- oder Alkylthioäther oder Alkyl- oder Aralkylester von Polyäthylenoxidglykolen mit 8 bis 22 C-Atomen in Alkylgruppen und 5 bis 25 Äthylenoxideinheiten oder Alkyldimethylbenzyl- ammoniumchloridverbindungen mit 8 bis 22 C-Atomen in der Alkylgruppe,

in einer zum Auftreten einer Fällung des Komplexes ausreichenden Menge zugesetzt und anschließend wird weiteres Detergens zugesetzt, bis der ausgefallene Komplex wieder gelöst ist.

EP 0 004 857 A2

- 1 -

## Verfahren und Mittel zur Beseitigung von Trübungen

Die Erfindung betrifft ein Verfahren und ein Reagens zur Beseitigung von Trübungen in durch Probenmaterial, vorzugsweise biologisches Probematerial, z.B. Serum oder Plasma getrübten Reaktionsgemischen. Diese werden unter anderem für die klinisch-chemische Analytik, für die lebensmittelchemische Analytik sowie für die Wasser- und Abwasseranalytik verwendet. Zur Erreichung einer höheren Spezifität werden dabei häufig Enzyme eingesetzt.

Ein bekanntes Problem der photometrischen Analyse ist der Einfluß von Trübungen der Probeflüssigkeit, wie Serum oder Plasma, auf das Analysenergebnis, wenn die Messung ohne Probenleerwert erfolgt. Die Vermeidung der Messung eines Probenleerwerts wird angestrebt, um den Reagensverbrauch zu vermindern, den Meß- und Auswertevorgang zu vereinfachen und den Probeneinsatz zu verringern. Bei Verwendung von Eintopfreagentien, die alle reaktiven Komponenten gemischt enthalten, ist die Messung eines Probenleerwerts sogar prinzipiell unmöglich. Derartige Trübungen sind durch verschiedene Ursachen bedingt, häufig durch hohen Triglyceridgehalt.

Eine Trübung führt zu einer erhöhten Eigenextinktion im Probe-Reagens-Gemisch. Da sich die gesamte Extinktion aus dem Farbanteil, der der Konzentration des zu bestimmenden Substrats direkt proportional ist, und aus einem Trübungsanteil zusammensetzt, erhält man notwendigerweise hierbei falsche Ergebnisse.

0004857

Es ist bekannt, solche Trübungen durch den Zusatz von Detergentien soweit aufzuhellen, daß der Trübungsanteil an der Gesamtextinktion vernachlässigt werden konnte (DE-AS 2 327 894).

Ein wesentlicher Nachteil dieses Verfahrens liegt darin, daß sehr hohe Detergenzmengen benötigt werden. Die erforderlichen hohen Konzentrationen sind nachteilig, da hierbei häufig die Aktivität eingesetzter Enzyme verringert wird und außerdem derartige Mengen sich nicht mehr in Feststoffgemischen unterbringen lassen, die trocken und rieselfähig sein sollen. Dies ist besonders bei Eintopfreagentien von Bedeutung.

Der Erfindung liegt die Aufgabe zugrunde, die oben erwähnten Nachteile zu beseitigen und ein Verfahren und ein Reagens zu schaffen, welches es gestattet, die oben erläuterten Trübungen zu beseitigen. Unter Beseitigung wird dabei im Rahmen der Erfindung auch die Verhinderung oder Verminderung derartiger Trübungen verstanden.

Gelöst wird diese Aufgabe erfindungsgemäß durch ein Verfahren zur Beseitigung von Trübungen in durch Probematerial getrübten Reaktionsgemischen, insbesondere für photometrische Messung, durch Zusatz von Detergentien, welches dadurch gekennzeichnet ist, daß dem Reaktionsgemisch

a) eine oder mehrere niedermolekulare organische Verbindungen und

b) ein oder mehrere für sich klar lösliche Detergentien, welche mit dem Zusatz a) einen weniger löslichen Komplex bilden,

in einer zum Auftreten einer Fällung des Komplexes ausreichenden Menge zugesetzt werden und anschließend weiteres Detergens zugesetzt wird, bis der ausgefallene Komplex wieder gelöst ist.

Die Erfindung beruht auf der überraschenden Feststellung der Tatsache, daß die genannten organischen Verbindungen, die für sich allein in einer klaren wäßrigen Lösung keinerlei oder nur sehr geringe aufhellende Wirksamkeit auf die oben beschriebenen Trübungen zeigen, die an sich bereits bekannte Aufhellungswirkung von Detergentien synergistisch außerordentlich verstärken. Dies führt dazu, daß in den meisten Fällen nicht nur eine Aufhellung ermöglicht wird, sondern die Trübung ganz beseitigt werden kann, wobei gleichzeitig auch die hierfür erforderliche Detergensmenge drastisch verringert wird. Beispiele für die unter a) einsetzbaren Verbindungen sind:

a) aromatische (kondensierte und nichtkondensierte) Alkohole und Amine, bevorzugt solche, die stark elektronegativ substituiert sind (bevorzugte elektro-negative Substituenten sind Halogene), also z.B. Benzylalkohol, Phenol, Mono-, Di- und Trichlorphenole, Dibrom- und Tribromphenole, Kresole, Naphthole, Dihydroxynaphthole, Anilin und Chloranilin, Methylanilin;

b) Ester von Arylsäuren und Alkylsäuren, also z.B. p-Hydroxy-benzoesäureäthylester, Essigsäureäthylester, Essigsäurebutylester, Propionsäureäthylester, Trichloressigsäureäthylester und ähnliche;

c) geradkettige und cyclische und verzweigte aliphatische Alkohole mit mehr als 3 C-Atomen, also z.B. Amylalkohol, Octanol, Dodekanol, Cyclohexanol und ähnliche;

d) halogenierte kurzkettige aliphatische Verbindungen, also z.B. Dichloräthan, Trichloräthylen, Tetrachlormethan, Dichlorbuten, Tetrachlordifluoräthan und ähnliche;

e) aliphatische Ketone, also z.B. Butanon-(2), Cyclohexanon und ähnliche;

f) Aldehyde, wie z.B. Zimtaldehyd oder Benzaldehyd;

g) Carbonsäuren, z.B. Octansäure;

h) Äther, wie z.B. 2-Methoxyphenol;

i) Benzol.

Die jeweilige Menge einer solchen Verbindung (im folgenden Adjuvans genannt) für einen bestimmten Fall läßt sich durch Vorversuche ermitteln. Im allgemeinen wurde gefunden, daß Konzentrationen zwischen 0,5 und 300 mM, vorzugsweise zwischen 2 und 10 mM, gute Ergebnisse bringen.

Unter den mit den oben genannten Adjuvantien wenig lösliche Komplexe bildenden Detergentien, die andererseits alleine im Reaktionsgemisch klar löslich sind, finden sich Vertreter der nichtionigen, ionogenen und der amphoteren Detergentien. Bevorzugt werden die nichtionogenen Polyäthylenoxidaddukte. Besonders gute Ergebnisse werden erzielt mit Alkyl-, Aralkyl- und Alkylthioäthern sowie Alkyl- und Aralkylestern von Polyäthylenoxid-glykolen. In diesen Detergentien weisen die Alkylgruppen im allgemeinen 8 bis 22 C-Atome auf, die Arylgruppe ist vorzugsweise ein Phenylrest. Im Glykolrest dieser Detergentien liegen im allgemeinen 3 bis 25 Äthylenoxideinheiten in kondensierter Form vor. Typische Beispiele für diese besonders bevorzugte Gruppe von Detergentien sind Thesit, Genapol, Tergitol, Triton X100, Lensodel und Brij 35. Daneben können aber auch ionische Detergentien verwendet werden, insbesondere Alkyldimethylbenzyl-ammoniumchloride. Für die Alkylgruppe gilt das oben bei den Polyäthylenoxidaddukten Ausgeführte. Auch sekundäre Alkylsulfate, deren Alkylgruppen der obigen Definition entsprechen, erwiesen sich als brauchbar. Von den amphoteren Detergentien erwies sich N-Lauryldiäthanolamid als geeignet.

Im Rahmen der Erfindung wird, wie bereits erwähnt, die erforderliche Detergensmenge gegenüber dem Stand der Technik außerordentlich erniedrigt. Im allgemeinen reichen Mengen zwischen 0,05 und 1 % aus. Bevorzugt werden Detergentienzusätze zwischen 0,2 und 0,8 %. Es können natürlich auch höhere Konzentrationen angewendet werden,

0004857

hier steigt jedoch die Gefahr einer negativen Beeinflussung der eingesetzten Enzyme. Demgegenüber werden nach der oben angegebenen DE-AS 10 Vol.-% Detergens für die Aufhellung eingesetzt.

Besonders niedrige Detergenskonzentrationen können zur Anwendung kommen, wenn die Auflösung des Komplexes aus Detergens und Adjuvans durch Zusatz eines Detergens erfolgt, welches von dem im Komplex enthaltenen Detergens verschieden ist. Mit anderen Worten werden bei dieser bevorzugten Ausführungsform der Erfindung wenigstens zwei verschiedene Detergentien eingesetzt. Dabei kann als zweites Detergens auch ein Detergens verwendet werden, welches mit dem Adjuvans keinen schwerlöslichen Komplex zu bilden vermag, der sich durch eine Trübung erkennen läßt.

Ein weiterer Gegenstand der Erfindung ist ein Aufhellungsmittel für durch Probematerial getrübte Reaktionsgemische für Analysenverfahren, insbesondere für die photometrische Messung, mit einem Gehalt an Detergens, welches dadurch gekennzeichnet ist, daß es
a) aus einer oder mehreren niedermolekularen organischen Verbindungen und
b) einem oder mehreren ebenfalls für sich klar löslichen Detergentien, welche mit dem Bestandteil a) einen weniger löslichen Komplex bilden und gegebenenfalls
c) einem oder mehreren weiteren, von b) verschiedenen Detergentien, welche mit a) keinen wenig löslichen Komplex zu bilden brauchen,
besteht oder diese enthält.

Für dieses Aufhellungsmittel gelten die obigen Ausführungen zu Art und Menge der verwendeten Detergentien und Adjuvantien sinngemäß.

Das Verfahren ist anwendbar im Zusammenhang mit allen Arten von Messungen, bei denen auftretende Trübungen zu einer Verfälschung oder Störung der Messung führen können. Insbesondere handelt es sich hierbei um Reaktionsgemische, die für die photometrische Messung bestimmt sind. Derartige Reaktionsgemische sind dem Fachmann allgemein bekannt und werden beispielsweise für viele zu bestimmende Substanzen in "Methoden der enzymatischen Analyse", von H.U. Bergmeyer, Verlag Chemie, Weinheim/Bergstraße, beschrieben. Eine nähere Erläuterung derartiger Reaktionsgemische erübrigt sich daher hier.

Beispiele 1 bis 36

Trübungsaufhellende Systeme

Nachstehende erfindungsgemäße Kombinationen ergaben innerhalb 1 Minute bis maximal 2 Stunden eine Beseitigung der Trübung im System.

2 ml Testgemisch + 2 μl Trinkmilch mit 3,5 % Fettgehalt 1 cm Schichtdicke, 546 nm, 25°C.

a) Variation des Adjuvans

In 0,1 m Tris/HCl-Puffer mit 2 % $MgSO_4 \cdot 7 H_2O$

| Beispiel Nr. | Stoff | Konzentration | Detergens- konz. 1) | pH |
|---|---|---|---|---|
| 1 | 2,4-Dichlorphenol | 8; 6; 4 mM | 5,6‰ | 8,0 |
| 2 | 2,3-Dichlorphenol | 8; 6; 4 mM | 2,8‰ | 8,0 |
| 3 | 2,4-Dibromphenol | 8; 4mM | 7,3‰ | 8,0 |
| 4 | Phenol | 1%; 0,5 % | 3,3% | 8,0 |
| 5 | 2,4,6-Trichlor- phenol | 24; 12; 8 mM | 7 ‰ | 7,1 |

| Beispiel Nr. | Stoff | Konzentration | Detergens-konz. 1) | pH |
|---|---|---|---|---|
| 6 | 1,7-Dihydroxy-naphthol | 8; 4 mM | 4,5 ‰ | 8,0 |
| 7 | 2,7-Dihydroxy-naphthol | 8; 4mM | 3,5 ‰ | 8,0 |
| 8 | 1,5-Dihydroxy-naphthol | 8; 6; 4 mM | 5,5 ‰ | 8,0 |
| 9 | Hydrochinon | 2 % | 30 ‰ | 8,0 |
| 10 | Anilin | 2;1,5; 1 Vol% | 15 ‰ | 8,0 |
| 11 | 2-Chloranilin | 0,8 Vol% | 17 ‰ | 8,0 |
| 12 | o-Toluidin | 1; 0,5 Vol% | 16 ‰ | 8,0 |
| 13 | Pentanol-(1) | 2 Vol% | 9 ‰ | 8,0 |
| 14 | Chloroform | 0,4 Vol% | 4 ‰ | 8,0 |
| 15 | Benzol | 0,75 Vol% | 30 ‰ | 8,0 |
| 16 | Guajakol | 1 Vol% | 7 ‰ | 8,0 |
| 17 | Benzaldehyd | 1 Vol% | 1,4 ‰ | 8,0 |
| 18 | Zimtaldehyd | 0,5 Vol% | 20 ‰ | 8,0 |
| 19 | Cyclohexanon | 3 Vol% | 1,2 ‰ | 8,0 |
| | (mit 6,0 % $K_2SO_4$ und 2,5 ‰ Genapol Ox-80) in 0,5m Kaliumphosphatpuffer | | | |
| 20 | Octansäure Na-Salz | 1 ‰ | 2,5 ‰ | 5,0 |

1) Hydroxypolyäthoxydodekan (Thesit)

b) Variation der Detergentien

In 0,1m Tris/HCl-puffer (pH 8,0) mit 2 % $MgSO_4 \cdot 7 H_2O$ und 8 mM/l 2,4-Dichlorphenol als Zusatz wurde mit folgenden Systemen gute Aufhellung erzielt:

| Beispiel Nr. | Detergens I | Detergens II | konz. |
|---|---|---|---|
| 21 | Thesit 2) | - | 5,6 ‰ |
| 22 | Tergitol NPX 3) | - | 10 ‰ |
| 23 | Triton X100 4) | - | 17 ‰ |
| 24 | Pegosperse 5) | - | 15 ‰ |

| Beispiel Nr. | Detergens I | Detergens II | konz. |
|---|---|---|---|
| 25 | Thesit | Brij 35 | 2 %o/2 %o |
| 26 | Thesit | Tween 20 | 2 %o/1 %o |
| 27 | Thesit | Benzalkonium-chlorid | 3,5 %o/ 0,8 %o |
| 28 | Teepol 710 6) | Brij 35 | 5 %o/7 %o |
| 29 | Lensodel AB6 7) | Brij 35 | 5 %o/12 %o |
| 30 | Thesit | Cetyltrimethyl-ammoniumbromid | 4 %o/1 %o |
| 31 | Tween 20 8) | Laurinsäure-bis-(2-hydroxyäthyl)-amin | 8 %o/2 %o |
| 32 | Brij 35 9) | Laurinsäure-bis-(2-hydroxyäthyl)-amin | 10 %o/2 %o |
| 33 * | Genapol Ox80 10) | Dodecylsulfat-Na-Salz | 3 %o/16 %o |
| (in Anwesenheit von $K^+$-Ionen) | | | |
| 34 | Tween 20 | Cetyltrimethyl-ammoniumbromid | 7,5 %o/1 %o |
| 35 | Genapol Ox80 | Tween 20 | 1 %o/3,5 %o |
| 36 * | Genapol Ox100 11) | Dodecylsulfat-Na-Salz | 6 %o/3 %o |

2) Siehe 1)

3) Tergitol NPX = 10,5 Äthoxy-nonylphenol

4) Triton X100 = 9-10 Äthoxy-octylphenol

5) Pegosperse = Polyäthylenglykol-laurylester

6) Teepol 710 ) gemischte sek. Alkylsulfate

7) Lensodel AB6 (mit $C_8$ bis $C_{18}$)

8) Tween 20 = 20 Äthoxy-sorbitan-monolaurat

9) Brij 35 = Polyäthylen-lauryläther

10) Genapol Ox80) Fettalkoholpolyglykoläther

11) Genapol X100) (geradkettig mit $C_{12}$ bis $C_{15}$)

Beispiel 37

Reagens zur Bestimmung von Harnsäure im Serum

Rezeptur: 0,05 Mol/l $K_4P_2O_7$

10 Vol.-% Äthanol

mit HCl auf pH 8,5 gestellt

1 mg/ml NADP

1 U/ml Alkoholdehydrogenase EC 1.2.1.5

1000 U/ml Katalase EC 1.11.1.6

0,05 Mol/l $Na_2$-Oxalat

0,05 Mol/l Pyrazol

Die vorgegebene Meßwellenlänge und der relativ hohe erforderliche Probeneinsatz bedingen, daß bei lipämisch trübem Probenmaterial die Messung erschwert bzw. unmöglich gemacht wird.

Zur Beseitigung dieser Trübung wurden folgende Zusätze vorgenommen:

1) 2 % Essigsäurebutylester

1,5 % Thesit

2) 0,5 % Dichloräthan

0,5 % Hexanol

5 %o Genapol X-80 (Isotridecanol-polyglykoläther)

9 %o Genapol X-100 (Isotridecanol-polyglykoläther)

6 %o Thesit

Testansatz: 2 ml Reagens (mit den Zusätzen 1) oder 2)

0,1 ml Probe

Start mit 0,6 U Uricase EC 1.7.3.3.

Messung bei 340 nm und 25°C auf Endpunkt.

Die obigen Zusätze bewirken, daß die durch lipämisch trübes Probenmaterial stark erhöhten Anfangsextinktionen

- 10 -

in kürzester Zeit abgebaut werden.

Eine Trübung von um Extinktion 2,0 wird innerhalb von ca. 10 Minuten auf ein stabiles Niveau von ca. Extinktion 0,2 gegenüber dem Reagensleerwert verringert. Dadurch wird es möglich, abzugleichen, um dann die Reaktion zu starten.

Beispiel 38

Reagens zur Bestimmung von Cholesterin im Serum

Rezeptur: 2,54 g/l $KH_2PO_4$
31,60 g/l $K_2HPO_4$
8 g/l $Na_2SO_4$
200 mg/l 4-Aminoantipyrin
282 mg/l Phenol
pH 7,90
3 U/ml Peroxidase EC 1.11.1.7
0,3 U/ml Cholesterin-esterase EC 3.1.1.13
0,3 U/ml Cholesterin-oxidase EC 1.1.3.6

Dieses Reagens ist als Eintopf-gemisch konzipiert und muß daher ohne Messung eines Probenleerwerts anwendbar sein. Die Erfüllung dieser Forderung wird durch folgende Zusätze auch bei stark lipämisch trübem Probenmaterial gewährleistet:
Trübungsaufhellende Zusätze:
1) *815 mg/l 3,4-Dichlorphenol
4 g/l Genapol Ox-100
3 g/l Natriumdesoxycholat
2) 2 g/l Heptanol
8 g/l Thesit
3) 1 g/l Essigsäureäthylester
7 g/l Thesit

4) bei pH 7,0 und 80 mM K$^+$/Na$^+$-Phosphat-puffer

1,5 g/l Parahydroxy-benzoesäureäthylester

4 g/l Thesit

5) * bei pH 7,50 in 0,2m Kaliumphosphat-puffer zusätzlich zu den zur Farbbildung benötigten 3 mMol/l Phenol weitere

35 mMol/l Phenol

1,5 g/l Genapol Ox-100

1,5 g/l Tween 20

1,0 g/l Natriumcholat


Ansatz: 2 ml Reagens + 20 µl Probe, 546 nm, 25°C.


Durch das Probenmaterial bedingte Trübungen von Extinktion $\geq$ 1,5 werden innerhalb der für die Reaktion benötigten Zeitspanne völlig oder auf ein vernachlässigbares Maß abgebaut.


Beispiel 39

Reagens zur Bestimmung von Glucose im Serum

Rezeptur: 14,9 g/l Na$_2$HPO$_4$

13,2 g/l KH$_2$PO$_4$

8,0 g/l Na$_2$SO$_4$

30 U/ml Glucoseoxidase EC 1.1.3.4

1,8 U/ml Peroxidase EC 1.11.1.7

0,2 U/ml Esterase EC 3.1.1.13

470 mg/l Penol

156 mg/l 4-Aminoantipyrin

bei pH 7,0


Trübungsaufhellende Zusätze:

1) *814 mg/l 3,4-Dichlorphenol

3,6 g/l Genapol Ox-100

3,0 g/l Tauroglyco-cholsäure

2) *814 mg/l 3,4-Dichlorphenol

   2,5 g/l Thesit

   3,9 g/l Tauroglyco-cholsäure

3) *in 0,2m Kaliumphosphat-puffer, pH 7,0 zusätzlich 1,35 g/l Phenol

   489 mg/l 3,4-Dichlorphenol

   3,0 g/l Genapol Ox-100

   2,8 g/l Natriumcholat

Die Messung erfolgt hier bei 578 nm. Wie schon in Beispiel 38 erläutert, bewirken auch hier die Zusätze, daß die durch Probenmaterial verursachten Trübungen im Testansatz in kürzester Zeit abgebaut werden.

Die aufgeführten Beispiele beinhalten solche, bei denen

a) ein oder mehrere für sich klar lösliche Detergentien, die mit dem Zusatzstoff einen weniger löslichen Komplex bilden können und solche, bei denen

b) zusätzlich zu a) wenigstens ein Detergens, welches nicht in der Lage ist, mit dem Zusatzstoff unter den Bedingungen im Reaktionsgemisch einen weniger löslichen Komplex zu bilden (gekennzeichnet mit *),

eingesetzt werden.

Patentansprüche

1. Verfahren zur Beseitigung von Trübungen in durch Probenmaterial getrübten Reaktionsgemischen, insbesondere für photometrische Messung, durch Zusatz von Detergentien, dadurch gekennzeichnet, daß dem Reaktionsgemisch

a) eine oder mehrere niedermolekulare organische Verbindungen und

b) ein oder mehrere ebenfalls für sich klar lösliche Detergentien, welche mit dem Zusatz a) einen weniger löslichen Komplex bilden,

in einer zum Auftreten einer Fällung des Komplexes ausreichenden Menge zugesetzt werden und anschließend weiteres Detergens zugesetzt wird, bis der ausgefallene Komplex wieder gelöst ist.

2. Verfahren nach Anspruch 1, dadurch gekennzeichnet, daß aromatische Alkohole oder Amine, die elektro-negative Substituenten tragen, als Komponente a) verwendet werden.

3. Verfahren nach Anspruch 2, dadurch gekennzeichnet, daß Phenole oder Naphthole verwendet werden, welche mit ein oder mehreren Halogenatomen oder/und Hydroxylgruppen substituiert sind.

4. Verfahren nach Anspruch 3, dadurch gekennzeichnet, daß ein Di- oder Trihalogenphenol oder Dihydroxynaphthol verwendet wird.

5. Verfahren nach einem der Ansprüche 1 bis 4, dadurch gekennzeichnet, daß als Detergentien nichtionogene Polyäthylenoxidaddukte oder kationische Detergentien verwendet werden.

6. Verfahren nach Anspruch 5, dadurch gekennzeichnet, daß Alkyl-, Aralkyl- oder Alkylthioäther- oder Alkyl- oder Aralkylester von Polyäthylenoxidglykolen mit 8 bis 22 C-Atomen in den Alkylgruppen und 5 bis 25 Äthylenoxideinheiten verwendet werden.

7. Verfahren nach Anspruch 5, dadurch gekennzeichnet, daß Alkyldimethylbenzyl-ammoniumchloridverbindungen mit 8 bis 22 C-Atomen in der Alkylgruppe verwendet werden.

8. Verfahren nach einem der vorhergehenden Ansprüche, dadurch gekennzeichnet, daß zur Auflösung des gebildeten Komplexes ein Detergens zugesetzt wird, welches von dem im ausgefallenen Komplex enthaltenen Detergens verschieden ist.

9. Aufhellungsmittel für durch Probenmaterial getrübte Reaktionsgemische für Analysenverfahren, insbesondere für die photometrische Messung, mit einem Gehalt an Detergens, dadurch gekennzeichnet, daß es
   a) aus einer oder mehreren niedermolekularen organischen Verbindungen und
   b) einem oder mehreren ebenfalls für sich klar löslichen Detergentien, welche mit dem Bestandteil a) einen weniger löslichen Komplex bilden und gegebenenfalls
   c) einem oder mehreren weiteren, von b) verschiedenen Detergentien, welche mit a) keinen wenig löslichen Komplex bilden brauchen,
   besteht oder diese enthält.

10. Mittel nach Anspruch 9, dadurch gekennzeichnet, daß es aromatische Alkohole oder Amine, die elektronegative Substituenten tragen, oder Gemische davon enthält.

11. Mittel nach Anspruch 9 oder 10, dadurch gekennzeichnet, daß es als Detergens einen Alkyl-, Aralkyl- oder Alkylthioäther oder Alkyl- oder Aralkylester eines Polyäthylenoxidglykols oder/und ein Alkyldimethylbenzyl-ammoniumchlorid oder Gemische davon enthält.